# EUROPEAN PATENT APPLICATION

(11) **EP 2 098 221 A1**
(43) Date of publication of application: **09.09.2009**
(21) Application number: 07831102.4
(22) Date of filing: 02.11.2007
(51) Int. Cl.: A61K 9/127, A61K 47/18, A61K 47/24, A61K 47/28, A61N 1/30

(54) **COMPOSITION FOR IONTOPHORESIS FOR DELIVERY THROUGH HAIR PORE**

(30) Priority: 02.11.2006 JP 2006299448
(71) Applicant: HOKKAIDO UNIVERSITY, Sapporo-shi, Hokkaido 060-0808 (JP); TTI ellebeau, Inc., Tokyo 140-0002 (JP)
(72) Inventor: KOGURE, Kentaro, Sapporo-shi, Hokkaido 060-0812 (JP); YAMAMOTO, Masahiko, Sapporo-shi, Hokkaido 060-0812 (JP); HARASHIMA, Hideyoshi, Sapporo-shi, Hokkaido 060-0812 (JP)
(74) Representative: Liesegang, Eva
(86) International application number: PCT/JP2007/071368
(87) International publication number: WO 2008/053983

(57) **Abstract**

The present invention relates to a composition which enables stable and efficient delivering of various active ingredients to deep regions of hair follicles and intradermal tissues in the vicinity of hair follicles. More specifically, the present invention relates to a composition for administering an active ingredient to a living body by iontophoresis, comprising
the active ingredient encapsulated in liposome, wherein
the liposome comprises: as constituent components, a cationic lipid; and an amphiphilic glycerophospholipid containing as constituent fatty acids both a saturated fatty acid and an unsaturated fatty acid.

## Description

### [Technical Field]

The present invention relates to a technology of transdermally administering various active ingredients by iontophoresis, and more particularly relates to a composition useful for stably and efficiently delivering various active ingredients to deep regions of hair follicles and intradermal tissues in the vicinity of hair follicles by iontophoresis.

### [Background of Art]

A method involving applying, to an ionic drug placed on the surface of the skin or the mucosa (hereinafter merely referred to as "skin") of a predetermined region of a living body, electromotive force which drives the ionic drug for introducing (impregnating) the drug into the living body through the skin is referred to as iontophoresis (see Patent Document 1).

For example, a positively charged ion is driven (transported) into the skin at an anode side of an electric system of an iontophoresis device. In contrast, a negatively charged ion is driven (transported) into the skin at a cathode side of the electric system of the iontophoresis device.

According to iontophoresis, the first-pass effect of liver can be avoided, and initiation, cessation, of administration of a drug can be easily controlled. Then, in recent years, iontophoresis has been drawing attention as an administration method aiming at not only a local action but also a systemic action.

In iontophoresis, it is common that a drug is administered to a living body mainly through the corneum of the skin. However, the corneum is a lipid-soluble high-density layer and, in many cases, high water-soluble substances and polymers such as peptides, nucleic acids, etc. are difficult to pass therethrough. Moreover, there is a problem that iontophoresis is inapplicable to the non-charged substances as they are.

In order to transdermally administer substances with various physico-chemical properties, applying charged liposome as a carrier to iontophoresis has been studied (Median VM et al., International Journal of Pharmaceutics, Dec. 8, 2005:306(1-2):1-14.Epub Nov. 2, 2005 Epub 2005 Nov 2. Review; Non-patent Document 1). However, liposome has a large particle diameter, which makes it difficult to pass through the corneum as it is.

In contrast, it is known that hair follicles, which are connected from the skin surface to a deep region of the skin, are targeted in order to transdermally administer liposome efficiently (e.g., Hoffman RT et al., Nat Med. 1995 Jul;1(7):705-706; Non-patent Document 2, Fleisher D et al, Life Sci. 1995;57 (13):1293-1297; Non-patent Document 3).

Further, in recent years, the administration of liposome through hair follicles has been studied also in iontophoresis. For example, Protopapa EE et al. reported that liposome enclosing an enzyme is delivered to hair follicle stem cells in hair follicles by iontophoresis (Protopapa EE et al., J Eur Acad Dermatol Venereol. 1999 Jul;13(1):28-35; Non-patent Document 4).

Han I et al. reported that liposome encapsulating 5-aminolevulinic acid as an agent for a photo dynamic therapy is delivered to the hair follicle sebaceous gland and the like in upper regions of hair follicles by iontophoresis (Han I et al., Arch Dermatol Res. 2005 Nov;295(5):210-217. Epub 2005 Nov 11; Non-patent Document 5).

Han I et al. also reported that liposome encapsulating adriamycin as an agent for treating hair follicle-associated tumors is delivered to hair follicles by iontophoresis (Han I et al., Exp Dermatol. 2004 Feb;13(2):86-92; Non-patent Document 6).

In conventional iontophoresis targeting hair follicles, skin associated diseases and the like are therapeutic targets, and the conventional iontophoresis mainly aims to deliver a drug to upper regions of skin tissues. However, in the case where a drug or the like is administered aiming at a systemic action by iontophoresis, it is desirable to deliver a drug to a general circulation system through a subcutaneous blood vessel which exists in deep regions of hair follicles. Moreover, in the case where antibody production induction is intended while targeting, for example, Langerhans' cells and the like which exist at intradermal tissues in the vicinity of hair follicles, it is requested that a drug such as vaccine is securely delivered to the intradermal tissues in the vicinity of hair follicles. Thus, an important object of the iontophoresis targeting hair follicles is to stably and efficiently deliver liposome enclosing a drug to deep regions of hair follicles and intradermal tissues in the vicinity of hair follicles.

### [Brief of the Invention]

The inventors of the present invention now acquired findings that, by encapsulating an active ingredient in liposome with a specific constitution for applying the liposome to iontophoresis, the active ingredient can be stably and efficiently delivered to deep regions of hair follicles and intradermal tissues in the vicinity of hair follicles. The present invention is based on these findings.

Therefore, the present invention aims to provide a composition capable of stably and efficiently delivering an active ingredient such as a drug to deep regions of hair follicles and intradermal tissues in the vicinity of hair follicles by iontophoresis.

A composition for administering an active ingredient to a living body by iontophoresis of the present invention includes:
the active ingredient encapsulated in liposome, in which the liposome includes: as a constituent component,
a cationic lipid; and
an amphiphilic glycerophospholipid containing as a constituent fatty acid both saturated fatty acid and unsaturated fatty acid.

Thus, since an active ingredient is encapsulated in liposome with a specific constitution in the composition of the present invention, the active ingredient can be stably and efficiently delivered to deep regions of hair follicles and intradermal tissues in the vicinity of hair follicles by iontophoresis.

### [Brief Description of the Drawings]

FIG. 1 is a view schematically illustrating an in vitro skin penetration test using an iontophoresis device of the present invention.
FIGS. 2 are CLSM photographs (x10) of a fluorescence of NBD (4-chloro-1-nitrobenzofrazan) in a liposome outer layer of the present invention, the liposome which was administered to a rat skin in vitro.
FIGS. 3 are CLSM photographs (x10) of a fluorescence of Sulfo rhodamine B in an inner layer in the liposome of the present invention, the liposome which was administered to the rat skin in vitro.
FIGS. 4 are CLSM photographs (x10) of a fluorescence of Sulfo rhodamine B which was administered to the rat skin in vitro.
FIG. 5 is a view schematically illustrating an in vivo skin penetration test using the iontophoresis device of the present invention.
FIGS. 6 are CLSM photographs (x10) of the fluorescence of Sulfo rhodamine B in the inner layer in the liposome of the present invention, the liposome which was administered to the rat skin in vivo.
FIGS. 7 are CLSM photographs (x10) of the fluorescence of Sulfo rhodamine B which was administered to the rat skin in vivo.

### [Disclosure of the Invention]

In this specification, unless otherwise specified, "C" in a group or a part of a group refers to "total carbon number" in a group or a part of a group. Thus, for example, "C₁₋₆ saturated fatty acid" refers to "saturated fatty acid whose total carbon number is 1 to 6", and "C₁₂₋₃₁ cholesteryl fatty acid ester" refers to cholesteryl fatty acid ester containing "fatty acid whose total carbon number is 12 to 31".

Moreover, the terms "alkyl", "alkenyl", or "alkynyl" as a group or a part of a group refers to straight chain, branched chain, or cyclic alkyl, alkenyl, or alkynyl, and preferable are straight chain alkyl, alkenyl, or alkynyl or branched chain alkyl, alkenyl, or alkynyl, and more preferable are straight chain alkyl, alkenyl, or alkynyl are.

In the "cholesteryl fatty acid" or "dihydrocholesteryl fatty acid", the fatty acid may be saturated or unsaturated. The above-mentioned fatty acid may be straight chain, branched chain, or cyclic fatty acid. The fatty acid in the "cholesteryl fatty acid" is preferably straight chain fatty acid, and the fatty acid in the "dihydrocholesteryl fatty acid" is preferably straight chain fatty acid.

Unless otherwise specified, "aryl" refers to phenyl or naphthyl. The term "heteroaryl" refers to, unless otherwise specified, 5- to 6- membered heteroaryl (5- to 6- membered cyclic aromatic heterocyclic group) containing 1 to 3 nitrogens, oxygens, or sulfur atoms.

The "front surface" refers to a side near the skin of a living body on the path of electric current flowing through the inside of the electrode structure in administering liposome.

### Composition for Iontophoresis

As described above, the composition of the present invention contains an active ingredient encapsulated in liposome, in which the liposome contains, as a constituent component, cationic lipid and an amphiphilic glycerophospholipid including both of saturated fatty acid and unsaturated fatty acid as a constituent fatty acid. It is an unexpected fact that liposome containing such specific constituent components is advantageous to stably deliver an active ingredient to deep regions of hair follicles and intradermal tissues in the vicinity of hair follicles by iontophoresis.

The cationic lipid as a constituent component of liposome in the present invention is preferably a C₁₋₂₀ alkane substituted with a C₁₋₂₀ acyloxy group and a triC₁₋₄ alkylammonium group. Further, the above-mentioned C₁₋₂₀ alkane is preferably C₁₋₅ alkane, and more preferably C₁₋₃ alkane.

The above-mentioned C₁₋₂₀ alkane contains, as a substituent, preferably one to four C₁₋₂₀ acyloxy groups, and more preferably two C₁₋₂₀ acyloxy groups. The above-mentioned C₁₋₂₂ acyloxy groups are preferably C₁₋₂₀ acyloxy groups and more preferably C₁₋₁₈ acyloxy groups.

In addition, specific examples of the C₁-C₂₂ acyloxy group include an alkyl carbonyloxy group, an akenyl carbonyloxy group, an alkynyl carbonyloxy group, an aryl carbonyloxy group, or heteroaryl carbonyloxy group. An alkyl carbonyloxy group, an akenyl carbonyloxy group, and an alkynyl carbonyloxy group are preferred. An akenyl carbonyloxy group is more preferred.

The above-mentioned C₁₋₂₀ alkane contains, as a substituent, preferably one to four triC₁₋₆ alkylammonium groups, and more preferably one triC₁₋₆ alkylammonium group. The above-mentioned triC₁₋₆ alkylammonium groups are preferably triC₁₋₄ alkylammonium groups. Examples of counter ion of the above-mentioned trialkylammonium group are, but not limited to, chlorine ion, bromine ion, iodine ion, fluorine ion, sulfurous ion, nitrous ion, etc., and preferable are chlorine ion, bromine ion, and iodine ion.

Specific examples of the cationic lipid include preferably 1,2-dioleoyloxy-3-trimethylammonium propane (DOTAP), dioctadecyldimethylammonium chloride (DODAC), N-(2,3-dioleyloxy)propyl-N,N,N-trimethylammonium (DOTMA), didodecylammonium bromide (DDAB), 1,2-dimyristoyloxypropyl-3-dimethylhydroxyethylammonium (DMRIE), and 2,3-dioleyloxy-N-[2(sperminecarboxamido)ethyl]-N,N,-dimethyl-1-propanaminum trifluoroacetate (DOSPA), and more preferably DOTAP.

The amphiphilic glycerophospholipid of the present invention contains both saturated fatty acid and unsaturated fatty acid as a constituent fatty acid. The amphiphilic glycerophospholipid includes phosphatidylcholine, phosphatidylethanolamine, phosphatidylglycerol, phosphatidic acid, cardiolipin, phosphatidylserine, phosphatidylinositol, etc., and phosphatidylcholine is preferable, and egg-yolk phosphatidylcholine is more preferable.

As saturated fatty acid among the constituent fatty acids of the amphiphilic glycerophospholipid, C₁₂₋₂₂ saturated fatty acid is preferable, and C₁₄₋₁₈ saturated fatty acid is more preferable. Specifically, saturated fatty acid is preferably at least one selected from the group consisting of palmitic acid, lauric acid, myristic acid, pentadecylic acid, margaric acid, stearic acid, tuberculostearic acid, arachidic acid, and behenic acid, and more preferably are palmitic acid, myristic acid, pentadecylic acid, margaric acid, or stearic acid.

Moreover, among the constituent fatty acids of the amphiphilic glycerophospholipid, as unsaturated fatty acid, C₁₄₋₂₂ unsaturated fatty acid is preferable, and C₁₄₋₂₀ unsaturated fatty acid is more preferable. The above-mentioned unsaturated fatty acid contains preferably 1 to 6 carbon-carbon double bonds, and more preferably 1 to 4 carbon-carbon double bonds.

Specific examples of the unsaturated fatty acid preferably include at least one selected from the group consisting of oleic acid, myristoleic acid, palmitoleic acid, elaidic acid, vaccenic acid, gadoleic acid, erucic acid, nervonic acid, linoleic acid, α-linoleic acid, eleostearic acid, stearidonic acid, arachidonic acid, eicosapentaenoic acid, clupanodonic acid, and docosahexaenoic acid. Oleic acid, myristoleic acid, palmitoleic acid, elaidic acid, vaccenic acid, gadoleic acid, erucic acid, nervonic acid, linoleic acid, α-linoleic acid, eleostearic acid, stearidonic acid, and arachidonic acid are more preferred.

According to another preferred embodiment of the present invention, in the amphipathic glycerophospholipid, the saturated fatty acid includes at least one selected from the group consisting of palmitic acid, myristic acid, pentadecylic acid, margaric acid, and stearic acid, and the unsaturated fatty acid includes at least one selected from the group consisting of oleic acid, myristoleic acid, palmitoleic acid, elaidic acid, vaccenic acid, gadoleic acid, erucic acid, nervonic acid, linoleic acid, α-linoleic acid, eleostearic acid, stearidonic acid, and arachidonic acid.

According to a more preferable aspect of the present invention, liposome further contains a sterol as a constituent component. The sterol in the present invention is preferably at least one selected from the group consisting of cholesterol, C₁₂₋₃₁ cholesteryl fatty acid, C₁₂₋₃₁ dihydrocholesteryl fatty acid, polyoxyethylene cholesteryl ether, and polyoxyethylene dihydrocholesteryl ether, and more preferably at least one selected from the group consisting of cholesterol, cholesteryl lanolate, cholesteryl oleate, cholesteryl nonanate, cholesteryl macadaminate, and dihydrocholesterol polyethylene glycol ether (specifically,DIHYDROCHOLETH-30 is mentioned). Cholesterol is still more preferable.

In some embodiments, the liposome of the present invention preferably contains, as a constituent component, cationic lipid, sterol and an amphiphilic glycerophospholipid including both of saturated fatty acid and unsaturated fatty acid as a constituent fatty acid.

In some embodiments, the liposome may contain a C₁₋₂₀ alkane substituted with a C₁₋₂₀ acyloxy group and a triC₁₋₄ alkylammonium group, sterol, and an amphiphilic glycerophospholipid containing both C₁₂₋₂₂ saturated fatty acid and C₁₄₋₂₂ unsaturated fatty acid having 1 to 6 carbon-carbon double bonds.

In some embodiments, the liposome may contain 1,2-dioleoyloxy-3-trimethylammonium propane; sterol at least one selected from the group consisting of cholesterol, cholesteryl lanolate, cholesteryl oleate, cholesteryl nonanate, cholesteryl macadaminate, and dihydrocholesterol polyethylene glycol ether; and amphiphilic glycerophospholipid containing both C₁₂₋₂₂ saturated fatty acid and C₁₄₋₂₂ unsaturated fatty acid having 1 to 6 carbon-carbon double bonds.

In some embodiments, the liposome may contain 1,2-dioleoyloxy-3-trimethylammonium propane; sterol; and amphiphilic glycerophospholipid containing both saturated fatty acid and unsaturated fatty acid. The saturated fatty acid is at least one selected from the group consisting of palmitic acid, lauric acid, myristic acid, pentadecylic acid, margaric acid, stearic acid, tuberculostearic acid, arachidic acid, and behenic acid. The unsaturated fatty acid is at least one selected from the group consisting of oleic acid, myristoleic acid, palmitoleic acid, elaidic acid, vaccenic acid, gadoleic acid, erucic acid, nervonic acid, linoleic acid, α-linoleic acid, eleostearic acid, stearidonic acid, arachidonic acid, eicosapentaenoic acid, clupanodonic acid, and docosahexaenoic acid.

In some embodiments, the liposome may contain 1,2-dioleoyloxy-3-trimethylammonium propane; cholesterol, and amphiphilic glycerophospholipid containing both saturated fatty acid and unsaturated fatty acid. The saturated fatty acid is at least one selected from the group consisting of palmitic acid, lauric acid, myristic acid, pentadecylic acid, margaric acid, stearic acid, tuberculostearic acid, arachidic acid, and behenic acid. The unsaturated fatty acid is at least one selected from the group consisting of oleic acid, myristoleic acid, palmitoleic acid, elaidic acid, vaccenic acid, gadoleic acid, erucic acid, nervonic acid, linoleic acid, α-linoleic acid, eleostearic acid, stearidonic acid, arachidonic acid, eicosapentaenoic acid, clupanodonic acid, and docosahexaenoic acid.

In some embodiments, the amphiphilic glycerophospholipid is phosphatidylcholine. In some embodiments, the liposome may contain 1,2-dioleoyloxy-3-trimethylammonium propane, cholesterol and egg-yolk phosphatidylcholine.

In some embodiments, in the liposome of the present invention, a molar ratio of the cationic lipid and the amphiphilic glycerophospholipid is preferably 3:7 to 7:3, and more preferably 4:6 to 6:4 in view of the stability and delivery efficiency of the liposome in iontophoresis. When the liposome contains a sterol, a molar ratio of the cationic lipid and the sterol is preferably 3:7 to 7:3, and more preferably 4:6 to 6:4.
Moreover, in the liposome of the present invention, a molar ratio of the amphiphilic glycerophospholipid and the sterol is preferably 3:7 to 7:3, and more preferably 4:6 to 6:4. In the liposome of the present invention, a molar ratio of the cationic lipid and the total of the amphiphilic glycerophospholipid and the sterol is preferably 3:7 to 7:3, and more preferably 4:6 to 6:4. According to one aspect of the present invention, a molar ratio of the cationic lipid, the amphiphilic glycerophospholipid, and the sterol is about 2:1:1.

The average particle diameter of the liposomes of the present invention is preferably about 400 nm or more, and more preferably 400 to 1,000 nm. The average particle diameter of the liposomes can be confirmed by, for example, a dynamic□light□scattering method, a static light scattering method, an electron microscope observation method, and an atomic force microscope observation method.

The active ingredient of the present invention may be a hydrophobic substance or a water soluble substance and may be a non-charged substance or a charged substance insofar as it can be encapsulated in liposome. As the active ingredient of the present invention, not only low molecular weight compounds but also high molecular weight compounds such as nucleic acids, peptides, etc., can be used insofar as they can be encapsulated in liposome. Examples of preferable active ingredients include drugs (vaccine, hair-growth agents, hair restorers, hair removers, hormones, etc.), colorants, nucleic acids (DNA, RNA, PNA, etc.), peptides, proteins, enzymes, lipopolysaccharides, fungus compound, etc. As the examples of the above-mentioned fungus compound, cell wall fractions, fibrous structure fraction, pilus component fraction, glucosyl transferase (GTF) fraction, and protein antigen fraction are mentioned, and they can be used as an antigen. The amount of the active ingredient encapsulated in the liposome can be suitably determined in view of physico-chemical properties, dosage, etc., of the active ingredient.

As a method of producing the liposome and the composition containing the liposome of the present invention, the following methods can be used, for example. First, cationic lipid, amphiphilic glycerophospholipid, and, as required, sterol or the like are mixed in desired ratios in an organic solvent such as CHCl₃ to obtain a suspension. The suspension is distilled under reduced pressure, and the addition of an organic solvent and distillation under reduced pressure are repeated, thereby obtaining a lipid film. Next, to the lipid film, a buffer such as 10 to 50 mM HEPES (2-[4-(2-hydroxyethy)-lpiperazinyl]ethanesulfonic acid) or the like and a desired amount of active ingredient are added. The obtained mixed liquid is left standing at room temperature for 10 minutes for hydration, followed by sonication. The sonication is performed, for example, at room temperature at 85 W for 1 minute, but the conditions are not limited thereto. The mixed liquid is treated using a membrane filter, extruder, etc., to adjust the particle diameter, thereby obtaining liposome of the present invention. The obtained liposome is further mixed suitably with a pharmacologically acceptable carrier and the like, thereby obtaining a composition of the present invention.

There is no limitation on the pharmacologically acceptable carrier for use in the present invention insofar as the administration of liposome by iontophoresis is not hindered. For example, surfactants, lubricants, dispersants, buffers such as HEPES, preservatives, solubilizing agents, antiseptics, stabilizing agents, antioxidants, colorants, are mentioned. The composition of the present invention can be formed into a suitable dosage form as desired, insofar as the administration of liposome by iontophoresis is not hindered. However, considering efficient administration of liposome by iontophoresis, the composition is preferably formed into a solution or suspension with HEPES buffer and/or electrolyte mentioned later. The above-mentioned composition of the present invention can be applied to various uses according to types and properties of an active ingredient to be encapsulated in liposome. When a drug is used as the active ingredient, the composition of the present invention can be used as medicine. Therefore, another aspect of the present invention provides the use of liposome of the present invention for producing pharmaceutical compositions. The liposome of the present invention can be stably and efficiently delivered to deep regions and intradermal tissues of hair follicles, thereby the liposome of the present invention can be applied to treatment of local diseases and intradermal vaccine, and further advantageously utilized for treatment or the like of various diseases requiring a systemic action.

In another aspect of the present invention provides a method of administering an active ingredient to a living body by iontophoresis, the method at least including placing the composition of the present invention as it is on the skin surface of a living body, and applying electric current to the skin. In the above-mentioned method, the active ingredient encapsulated in the liposome in the composition is administered to a living body through hair follicles.

The composition of the present invention may be placed on the skin surface by, for example, applying the composition as it is to the skin, or placing, on the skin surface, an electrode structure of an iontophoresis device in which the composition is held. Electric current is applied to the liposome, as it is, of the present invention encapsulating the active ingredient, and the resultant may be used in iontophoresis. Such an aspect is also encompassed by the present invention.

When applying electric current, it is preferable that anode current be applied on an anode side of an electric system because liposome of the present invention is cationic. In applying electric current, the current value is preferably from 0.1 to 0.6 mA/Cm², more preferably from 0.3 to 0.5 mA/cm², and still more preferably about 0.45 A/cm². Moreover, a period of time of applying electric current is preferably from 0.5 to 1.5 hours, more preferably from 0.75 to 1.25 hours, and still more preferably about 1 hour.

The above-mentioned living body include preferably mammals, more preferably rats, human, guinea pigs, rabbits, mice, and pigs, and still more preferably human beings.

### Electrode Structure and Device for Iontophoresis

As described above, the composition of the present invention can be used by being held in the electrode structure for iontophoresis. Therefore, in another aspect of the present invention provides an electrode structure for administering an active ingredient to a living body by iontophoresis, the electrode structure holding the composition of the present invention. The liposome of the present invention is cationic, and the electrode structure is connected to the anode side of the electric system of the iontophoresis device. Therefore, the electrode structure of the present invention contains at least anode and an active ingredient reservoir holding the composition of the present invention. In the electrode structure, the above-mentioned active ingredient reservoir may be directly disposed on the front surface of the anode and other component such as an ion exchange membrane, may be disposed between the anode and the active ingredient reservoir insofar as the administration of liposome by iontophoresis is not hindered. Mentioned as the electrode structure containing such other component is, for example, an electrode structure containing at least an anode, an electrolyte reservoir for holding electrolyte disposed on the front surface of the anode, an anion exchange membrane disposed on the front surface of the electrolyte reservoir, and an active ingredient reservoir for holding the composition of the present invention. Further, on the front surface of the above-mentioned active ingredient reservoir, a cation exchange membrane may be disposed as desired.

Further, another aspect of the present invention provides an iontophoresis device containing the above-mentioned electrode structure. The iontophoresis device contains at least a power unit, an electrode structure holding the composition of the present invention, which is connected to the power unit, and an electrode structure as a counter electrode of the electrode structure. The structure of the electrode structure as a counter electrode is not limited insofar as the administration of liposome by iontophoresis is not hindered. For example, the electrode structure as a counter electrode may contain a cathode, an electrolyte reservoir for holding electrolyte disposed on the front surface of the cathode, and an ion exchange membrane disposed on the front surface of the electrolyte reservoir. The above-mentioned ion exchange membrane may be an anion exchange membrane or a cation exchange membrane, and preferable is an anion exchange membrane.

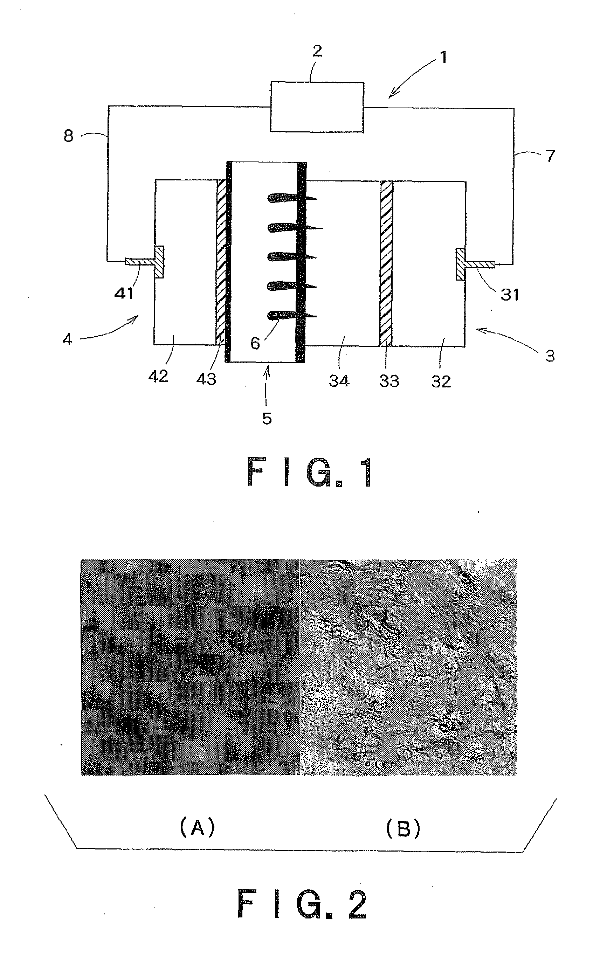

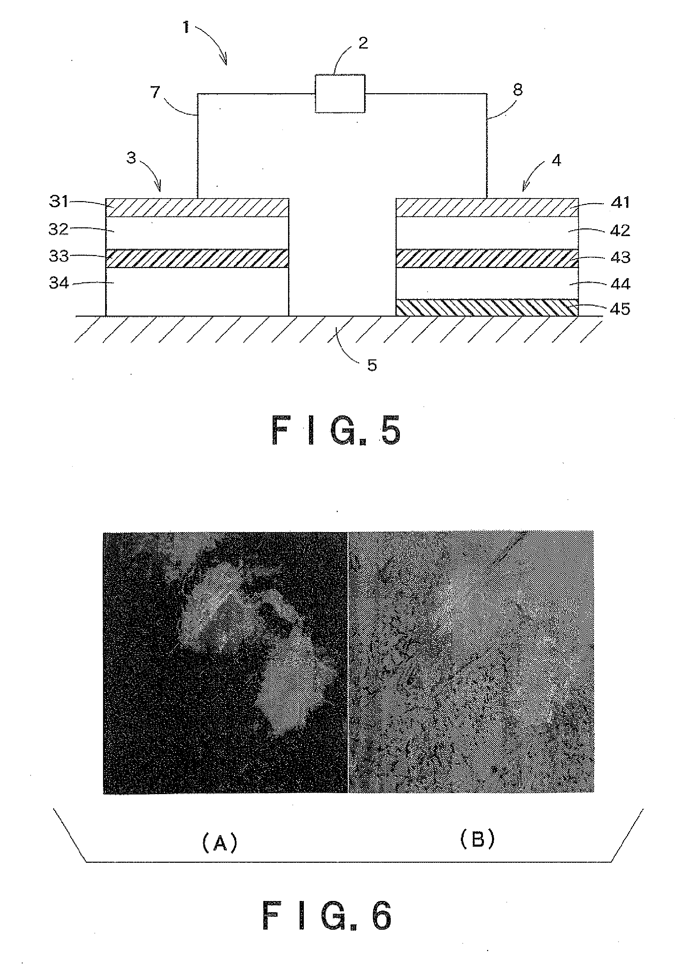

As a specific example of the above-mentioned electrode structure and the iontophoresis device of the present invention, an electrode structure and an iontophoresis device illustrated in Figures 1 and 5 and disclosed in International Publication WO 03/037425 A1 are, for example, specifically mentioned.

In the iontophoresis device of the present invention, liposome migrates to a side opposite to the positive electrode due to an electric field at the time of applying electric current, and is efficiently emitted from the electrode structure.
Therefore, another aspect of the present invention provides a method of operating an iontophoresis device, including:
disposing the electrode structure and a counter electrode of the electrode structure of the present invention on the skin surface of a living body; applying electric current to an iontophoresis device; and emitting liposome from the electrode structure of the present invention.

In the above-mentioned iontophoresis device, the active ingredient reservoir or the electrolyte reservoir may be formed of a cell (electrode chamber) which is, for example, formed of acryl and is filled with the composition of the present invention or electrolyte and may be formed of a thin membrane having properties of holding the composition of the present invention or electrolyte. With respect to the thin membrane, the same material can be used in the active ingredient reservoir and the electrolyte reservoir.

As the electrolyte, a desired electrolyte can be suitably used according to conditions of the active ingredient to be applied. However, electrolyte that adversely affects the skin of a living body due to electrode reaction should be avoided. As a suitable electrolyte in the present invention, an organic acid and salts thereof which exist in a metabolic cycle of a living body are preferable from the viewpoint of non-toxicity. For example, lactic acid and fumaric acid are preferable and, specifically, an aqueous solution in which a ratio of 1M lactic acid to 1M sodium fumarate is 1:1 is preferable.

It is important for the thin membrane forming the active ingredient reservoir to have sufficient ability to impregnate and retain a composition and electrolyte and to have sufficient ability to migrate ionized liposome impregnated and retained under predetermined electric field conditions to the skin side (ion transportation ability, ion electrical conductivity). As a material having both favorable impregnation and retaining properties, and favorable ion transportation ability, an acrylic resin hydrogel substance (acrylic hydrogel membrane), a segmented polyurethane gel membrane, an ion electorical-conductive porous sheet for forming a gel-like solid electrolyte (e.g., porous polymer disclosed in JP 11-273452 A which contains an acryl nitrile copolymer, as a base, having acrylonitrile in a proportion of 50 mol% or more, and preferably 70 to 98 mol% and having a porosity of 20 to 80%), or the like is mentioned. When impregnating the above-mentioned active ingredient reservoir, the impregnation degree (100x(WD)/D[%], wherein D represents a dry weight and W represents a weight after impregnation) is preferably 30 to 40%.

The conditions for impregnating the composition of the present invention or electrolyte into the active ingredient reservoir or the electrolyte reservoir are suitably determined according to the impregnation amount of electrolyte and an ionic drug, the impregnation rate, etc. The impregnation is performed, for example, at 40°C for 30 minutes.

As an electrode of the electrode structure, an inert electrode comprising, for example, an electrically conductive material such as carbon and platinum is preferably used.

As the ion exchange membrane used for the electrode structure, it is preferable to use a cation exchange membrane and an anion exchange membrane in combination. As the cation exchange membrane, NEOSEPTA® CM-1, CM-2, CMX, CMS, CMB, and CLE04-2 manufactured by Tokuyama Corporation, and the like are preferably mentioned. As the anion exchange membrane, NEOSEPTA® AM-1, AM-3, AMX, AHA, ACH, ACS, ALE04-2, and AIP-21 manufactured by Tokuyama Corporation, and the like are preferably mentioned. As another preferable example, a cation exchange membrane in which an ion exchange resin having a cation exchange function is impregnated in a part or whole of pores of a porous membrene or an anion exchange membrane in which an ion exchange resin having an anion exchange function is impregnated is mentioned.

Details of each of the above-mentioned constituent material are disclosed in International Publication WO 03/037425A1 filed by the applicant of the present invention, and the present invention includes the contents disclosed in this document.

### [Examples]

### Test Example 1

### Examination of Conditions of Applying Electric Current in Deliverin Liposome through Hair Follicle

### Preparation of Liposome

350 µL of CHCl₃ solution of 10 mM DOTAP (Avanti Polar Lipids, Inc.), 150 µL of CHCl₃ solution of 10 mM cholesterol (hereinafter referred to as "Chol", Avanti Polar Lipids, Inc.), and 6.4 µL of CHCl₃ solution of Rho-DOPE (1,2-dioleoyl-sn-glycero-3-phosphoethanolamine-N (lissamine rhodamine B sulfonyl)) were mixed. 500 µL of CHCl₃ was added to the mixture, thereby obtaining a suspension (molar ratio; DOTAP:Chol:Rho-DOPE=7:3:0.1). The suspension was distilled under reduced pressure using an evaporator, and then 400 µL of CHCl₃ was added, followed by distillation under reduced pressure again, thereby obtaining a lipid film. 1 mL of 10 mM HEPES buffer was added to the lipid film. The obtained mixed liquid was left standing at room temperature for 10 minutes for hydration, and then sonication (AU-25C ultrasonic cleaner, product of Aiwa Ika kogyo k.k.) was performed at room temperature at 85W for 1 minute. Further, the mixed liquid was treated using a PC membrane with a pore size of 400 nm and a PC membrane with a pore size of 100 nm (product name: Nuclepre Track-Etch Membrane, product of Whatman) by an extruder (product name: Mini-Extruder, product of Avanti Polar Lipids, Inc.), thereby obtaining a liposome suspension.

### Test of Applying Electric Current

The hair of the back of a SD rat (male, 10-weeks old, CLEA Japan, Inc.) was shaved, and the skin was collected. Next, 100 µL of the above-obtained liposome suspension was applied to the surface of the skin, and the liposomal suspension was spread on the skin. Next, as illustrated in FIG. 1, an iontophoresis device 1 equipped with a power unit 2, a working electrode structure 3, and a non-working electrode structure 4 as a counter electrode was placed on skin 5. Here, the working electrode structure 3 was placed on the front surface side of the skin 5 existing hair follicles 6; the non-working electrode structure 4 as a counter electrode was placed on the rear surface side of the skin 5; and both the electrode structures 3 and 4 were connected to the power unit through cords 7 and 8, respectively. The working electrode structure 3 contained a positive electrode 31, an electrolyte reservoir 32 holding 1 mL of electrolyte, the reservoir which was placed on the front surface of the positive electrode 31, an anion exchange membrane 33, and an active ingredient reservoir 34 holding 850 µL of liposome suspension. The non-working electrode structure 4 contained a negative electrode 41, an electrolyte reservoir 42 holding 1 mL of electrolyte, the reservoir which was placed adjacent to the negative electrode 41, and a cation exchange membrane 43.

The active ingredient reservoir 34 and the electrolyte reservoir (32, 42) employed an acrylic cell capable of retaining the active ingredient or the electrolyte in the interior space thereof. The above-mentioned anion exchange membrane 33 (product name: ALE04-2, product of Tokuyama Corporation) and the above-mentioned cation exchange membrane 43 (product name: CLE04-2, product of Tokuyama Corporation) were kept in physiological saline beforehand for use. Used as the above-mentioned electrolyte was electrolyte containing disodium fumarate (420 mM), L-ascorbic acid 2-phosphate trisodium salt (18.5 mM), and polyacrylic acid (0.4 mM).

Next, electric current was applied to the iontophoresis device 1 illustrated in FIG. 1 under various conditions indicated in Table 1 shown below. After the application of electric current, the skin 5 was removed from the iontophoresis device and the surface was wiped off with a filter paper. Then, the skin piece was collected and embedded in an OTC compound using liquid nitrogen. Further, a 15-µm-thick section was prepared from the obtained frozen block with a cryostat (CM3000, product of Leica), and fluorescence of rhodamine in the section was observed under a confocal laser scanning microscope (CLSM).

The conditions of applying electric current to the iontophoresis device 1 and the test results are shown in Table 1.
In the following table 1, ++ refers to a condition in which the delivery of liposome was observed in a deep region by 50% or more with respect to the length of a hair follicle, + refers to a condition in which the delivery of liposome was observed in a deep region within the range of 0 (hair follicle entrance) to less than 50% with respect to the length of a hair follicle, and - refers to a condition in which existence of liposome was not confirmed in hair follicles.
[Table 1]

**Table 1**

| | 1hr | 2hr | 3hr |
|---|---|---|---|
| 0.94mA(0.3mA/cm²) | - | + | ++ |
| 1.41mA(0.45mA/cm²) | ++ | ++ | - |
| 1.88mA(0.6mA/cm²) | - | - | - |

According to the results shown in Table 1, electric current was applied at 1.41 mA (0.45 mA/cm²) for 1 hour in the following tests.

### Test Example 2

### Examination of Composition of Lipid Liposome

### Preparation of Liposome

Liposomes of a lipid composition (molar ratio) shown in the following Table 2 were prepared. In the following, "EPC" refers to egg-yolk phosphatidylcholine (product of Nippon Yushi, Co., Ltd.), "DOPE" refers to 1,2-dioleoyl-sn-glycero-3-phosphoethanolamine (product of Avanti Polar Lipids, Inc.), "CHEMS" refers to Cholesteryl hemisuccinate (product of Avanti Polar Lipids, Inc.), and "DOTAP" and "Chol" are as mentioned above.
[Table 2]

**Table 2**

| Cationic liposome (lipid composition; molar ratio) | |
|---|---|
| (a) | DOTAP/Chol=7/3 |
| (b) | DOTAP/Chol=5/5 |
| (c) | DOTAP/EPC=5/5 |
| (d) | DOTAP/EPC=3/7 |
| (e) | DDTAP/DOPE=5/5 |
| (f) | DOTA/EPC/Chol=5/2.5/2.5 |
| (g) | DOTAP/EPC/Chol=5/4/1 |
| (h) | DOTAP/EPC/DOPE=5/2.5/2.5 |

| Anionic liposome (lipid composition ; molar ratio) | |
|---|---|
| (i) | CHEMS/EPC=2/9 |
| (j) | CHEMS/DOPE=2/9 |

In the preparation of liposomes, each lipid was mixed so that the molar ratio was as shown in Table 2, and 1 mol% Rhodamine-DOPE was added as a label. Then, liposome was obtained in the same manner as in Test Example 1. For example, in Table 2 (b), 350 µL of CHCl₃ solution of 10 mM DOTAP and CHCl₃ solution of 10 mM Chol were mixed. Further, 1 mol% Rhodamine-DOPE (6.4 µL) was added as a label, and then liposome was obtained in the same manner as in Test Example 1.

### Iontophoresis Test

Using liposome shown in Table 2, an iontophoresis test was performed under conditions of applying electric current at 1.41 mA (0.45 mA/cm²) for 1 hour. When using cationic liposomes shown in (a) to (h), Table 2, the iontophoresis device 1 illustrated in Figure 1 was used as it was. In contrast, when using anionic liposomes shown in (i) and (j), Table 2, used was an iontophoresis device in which, in the iontophoresis device 1 of Figure1, a cathod and a cation exchange membrane were disposed in place of the anode 31 and the anion exchange membrane 33 of the working electrode structure 3, and a anode and an anion exchange membrane were disposed in place of the cathode 41 and the cation exchange membrane 43 of the non-working electrode structure 4.

The results were as shown in Table 3. In Table 3, + refers to a condition in which the delivery of liposome was observed in a deep region within the range of 0 (hair follicle entrance) to less than 50% with respect to the length of a hair follicle, ++ refers to a condition in which the delivery of liposome was observed in a deep region by 50 to 75% with respect to the length of a hair follicle, +++ refers to a condition in which the delivery of liposome was observed in a deep region by 76 to 100% or more with respect to the length of a hair follicle, and - refers to a condition in which existence of liposome was not confirmed in hair follicles.
[Table 3]

**Table 3**

| Lipid composition | (a) | (b) | (c) | (d) | (e) | (f) | (g) | (h) | (i) | (j) |
|---|---|---|---|---|---|---|---|---|---|---|
| Impregnation ability into hair follicles | + | - | ++ | + | - | +++ | ++ | + | - | + |

As shown in Table 3, (c) containing DOTAP and EPC, and (f) and (g) each containing DOTAP, EPC, and Chol showed ++ and +++.

### Test Example 3

### Examination of Influence of Particle Diameter of Liposome on Delivery through Hair Follicle

### Preparation of Liposomes with Various Particle Diameters

Liposome (DOTA/EPC/Chol=5/2.5/2.5) having the same composition as that of (f) of Table 2 was prepared following the same procedure as in Test Example 1. Next, in accordance with a procedure described below, the particle diameter of liposome was adjusted.

### 3-a: Particle Diameter About 400 nm

After the mixed liquid was left standing at room temperature for 10 minutes for hydration in Test Example 1, the obtained lipid film was treated with a VORTEX (Tube mixer TRIO HM-2F, product of Asone) for 30 seconds, thereby preparing a liposome suspension with an average particle diameter of about 400 nm.

### 3-b: Particle Diameter About 250 nm

Following the procedure of 3-a, the mixed liquid was treated with an extruder using a 400-nm PC membrane, thereby obtaining a liposome suspension with an average particle diameter of about 200 nm. Then, the suspension was subjected to freezing treatment for 30 seconds using liquid nitrogen and subjected to thawing treatment at 40°C for 3 minutes, and this cycle was repeated three times, thereby preparing a liposome suspension with an average particle diameter of about 250 nm. The average particle diameter was confirmed by a dynamic light scattering method (Photal ELS-8000HO, product of Otsuka electronics).

### 3-c: Particle Diameter About 200 nm

The liposome suspension obtained in 3-a was treated with an extruder using a 400 nm PC membrane, thereby obtaining a liposome suspension with an average particle diameter of about 200 nm.

### 3-d: Particle Diameter About 150 nm

Following the procedure of Test Example 1 except for not treating with an extruder, a liposome suspension with an average particle diameter of about 150 nm was prepared.

### 3-e: Particle Diameter About 100 nm

Following the procedure of Test Example 1, a liposome suspension with an average particle diameter of about 100 nm was prepared.

### Iontophoresis test

Using the liposome suspensions 3-a to 3-e, iontophoresis was performed under the same conditions as those of Test Example 2.

The results were as shown in Table 4. In Table 4, + and +++ are similarly defined as in Table 3. Fluorescence of rhodamine was observed using a confocal laser scanning microscope (CLSM) under the same conditions as Test Example 1. The liposome with an average particle diameter of about 400 nm was delivered to deep regions of hair follicles, such as Bulge region.
[Table 4]

**Table 4**

| Average Particle Diameter (nm) | 100 | 150 | 200 | 250 | 400 |
|---|---|---|---|---|---|
| Impregnation ability into hair follicles | + | + | + | + | +++ |

### Test Example 4

### Confirmation Test of Delivery of Liposome in Vitro

### Preparation of Liposome

Liposome having an outer layer labeled with NBD (4-chloro-7-nitrobenzofrazan) and an inner layer labeled with Sulfo rhodamine B was prepared by the following procedure.

250 µL of CHCl₃ solution of 10 mM DOTAP, 125 µL of CHCl₃ solution of 10 mM EPC, 125 µL of CNCl₃ solution of 10 mM Chol, and 9.2 µL of CHCl₃ solution of NBD-DOPE were mixed. 500 µL of CHCl₃ was added to the mixture, thereby obtaining a suspension (DOTAP:BPC:Chol: NBD-DOPE=7:3:0.1). The suspension was distilled away under reduced pressure using an evaporator, and then 400 µL of CHCl₃ was added, followed by distillation under reduced pressure again, thereby obtaining a lipid film. 1 mL of HEPES buffer solution of 2.5 mM Sulfo rhodamine was added to the lipid film. The obtained mixed liquid was left standing at room temperature for 10 minutes for hydration, and then sonication (AU-25C ultrasonic cleaner, product of Aiwa Ika kogyo k.k.) was performed at room temperature at 85 W for 1 minute. Further, the mixed liquid was treated by an extruder (product name: Mini-Extruder, product of Avanti Polar Lipids, Inc.) using a PC membrane with a pore size of 400 nm and a PC membrane with a pore size of 100 nm (product name: Nuclepre Track-Etch Membrane, product of Whatman), thereby obtaining a suspension. The suspension was further subjected to ultracentrifugation at 20°C at 5300 rpm for 4 hours, and free Sulfo rhodamine was removed.

### Iontophoresis Test

Iontophoresis was performed using the above-obtained liposome suspension under the same conditions as those of Test Example 3. As control, an HEPES buffer solution of 2.5 mM Sulfo rhodamine was used. A photograph (x10) was taken with a confocal laser scanning microscope (CLSM; LSM510 META, product of Zeiss) using wavelengths λex=475 nm and λem=540 nm in detection of NBD labeling the outer layer of liposome, and using wavelengths λex=570 nm and λem=590 nm in detection of Sulfo rhodamine B.

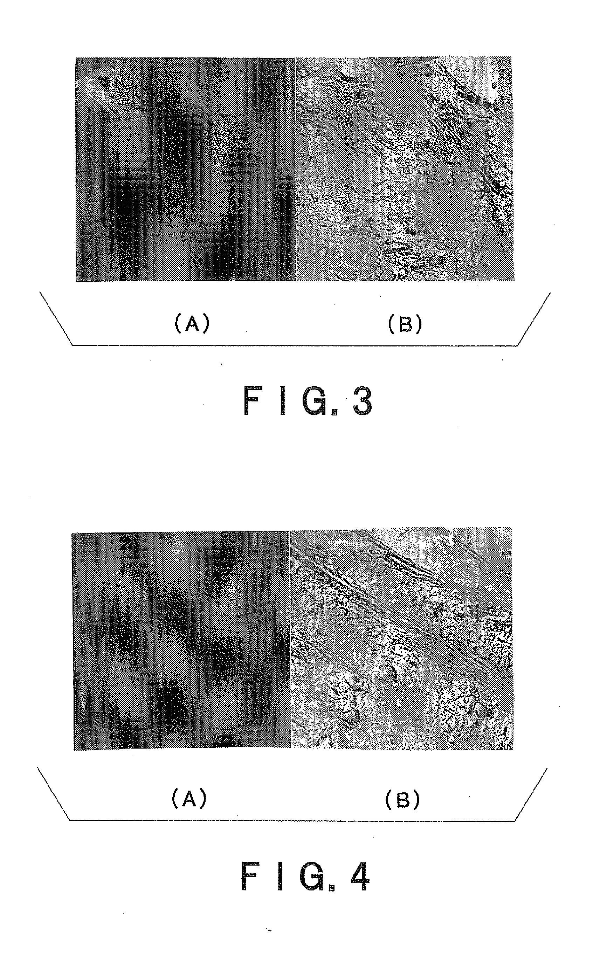

The results were as shown in Figures 2 to 4. In Figures 2 to 4, (A) is a photograph, taken with a fluorescence microscope, of a water-soluble fluorochrome (Rhodamine) and a fluorescence labeled lipid (NBD) in a dark field of the skin section and (B) is a photograph, taken with the microscope, of a bright field of the same skin section as that of (A). When the liposome suspension was used, NBD (Figures 2) labeling the outer layer of the liposome and Sulfo rhodamine B (Figures 3) labeling the inner layer thereof were detected at the same portion, and it was confirmed that the liposomes were delivered into hair follicles with their structures being retained. In contrast, Sulfo rhodamine B (Figures 4) as control was not detected in hair follicles, and it was confirmed that Sulfo rhodamine B was not delivered into hair follicles as it was.

### Test Example 5

### Confirmation Test of Delivery of Liposome through Hair Follicle Reparation of Liposome

A liposome suspension was prepared in the same manner as in Test Example 4.

### Iontophoresis Test using SD Rat

Anesthesia (ketamine / xylazine=10/1, 1 mg per weight kg) was administered to an SD rat (male, 10-weeks old, CLEA Japan, Inc.), and the hair of the back was shaved. Next, the iontophoresis device 1 containing the power unit 2, the working electrode structure 3, and the non-working electrode structure 4 was placed on the exposed skin 5 as illustrated in Figure 5. One hundred µL of the above-mentioned liposome suspension was applied beforehand to the contact surface of the exposed skin 5 and the working electrode structure 3. In the above-mentioned iontophoresis device 1, the working electrode structure 3 had the same structure as that of Test Example 1, i.e. the working electrode structure 3 had the anode 31, the electrolyte reservoir 32 holding 1 mL of electrolyte placed at the front surface of the anode 31, the anion exchange membrane 33, and the active ingredient reservoir 34 holding 850 µL of liposome suspension placed at the front surface of the anion exchange membrane 33. In contrast, the non-working electrode structure 4 had the cathode 41, the electrolyte reservoir 42 retaining 1 mL of electrolyte placed at the front surface of the cathode 41, the cation exchange membrane 43, an electrolyte reservoir 44 holding 800 µL of physiological saline placed at the front surface of the cation exchange membrane 43, and an anion exchange membrane 45 placed at the front surface of the electrolyte reservoir 44. The above-mentioned anion exchange membranes 33 and 45 (ALE04-2, product of Tokuyama Corporation) and the cation exchange membrane 43 (CLE04-2, product of Tokuyama Corporation) were kept in physiological saline beforehand for use.

Next, an iontophoresis test was performed under conditions of applying electric current at 1.41 mA (0.45 mA/cm²) for 1 hour. 3 hours after the completion of applying electric current, the SD rat was euthanized with carbon dioxide gas. Then, the skin 5 where the first electrode structure 3 was placed was collected, and a skin section was obtained in the same manner as in Test Example 1. With respect to the skin section, NBD labeling the outer layer of liposome and Sulfo rhodamine B were detected in the same manner as in Test Example 4.

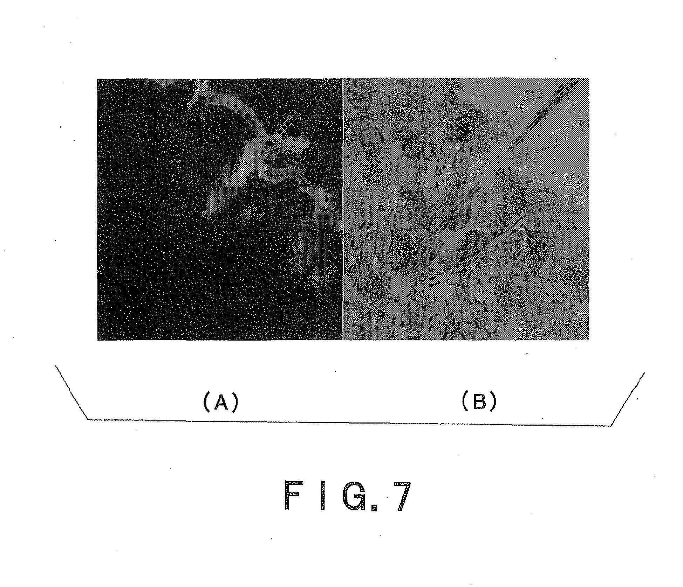

The results were as shown in Figures 6 and 7. In Figures 6 and 7, (A) is a photograph, taken with a fluorescence microscope, of a water-soluble fluorochrome (Rhodamine) and a fluorescence labeled lipid (NBD) in a dark field of the skin section and (B) is a photograph, taken with the microscope, of a bright field of the same skin section as that of (A). Also in vivo, the fluorescence of NBD (Figures 6) and the fluorescence of Sulfo rhodamine B (Figures 7) were detected at the same portion. In vivo test, it was confirmed that the liposomes were diffused from hair follicles to intradermal tissues with the structure being retained.

## Claims

1. A composition for administering an active ingredient to a living body by iontophoresis, comprising
the active ingredient encapsulated in liposome, wherein
the liposome comprises: as a constituent component,
a cationic lipid; and
an amphiphilic glycerophospholipid containing as a constituent fatty acid both saturated fatty acid and unsaturated fatty acid.

2. The composition according to claim 1, which is used for administering the active ingredient through a hair follicle.

3. The composition according to claim 1, wherein the cationic lipid is a C₁₋₂₀ alkane substituted with a C₁₋₂₂ acyloxy group and a triC₁₋₆ alkylammonium group.

4. The composition according to claim 3, wherein the cationic lipid comprises two C₁₋₂₂ acyloxy groups and one triC₁₋₆ alkylammonium group.

5. The composition according to claim 1, wherein the cationic lipid is 1,2-dioleoyloxy-3-(trimethylammonium)propane.

6. The composition according to claim 1, wherein the amphiphilic glycerophospholipid is phosphatidylcholine.

7. The composition according to claim 1, wherein the amphiphilic glycerophospholipid is an egg-yolk phosphatidylcholine.

8. The composition according to claim 1, wherein the saturated fatty acid is C₁₂₋₂₂ saturated fatty acid.

9. The composition according to claim 1, wherein the unsaturated fatty acid is at least one member selected from the group consisting of palmitic acid, lauric acid, myristic acid, pentadecanoic acid, margaric acid, stearic acid, tuberculostearic acid, arachidic acid, and behenic acid.

10. The composition according to claim 1, wherein the unsaturated fatty acid comprises 1 to 6 carbon-carbon unsaturated double bonds.

11. The composition according to claim 1, wherein the unsaturated fatty acid is C₁₄₋₂₂ unsaturated fatty acid.

12. The composition according to claim 1, wherein the unsaturated fatty acid is at least one member selected from the group consisting of oleic acid, myristoleic acid, palmitoleic acid, elaidic acid, vaccenic acid, gadoleic acid, erucic acid, nervonic acid, linolic acid, α-linoleic acid, eleostearic acid, stearidonic acid, arachidonic acid, eicosapentaenoic acid, clupanodonic acid, and docosahexaenoic acid.

13. The composition according to claim 1, wherein the liposome further comprises a sterol as a constituent component.

14. The composition according to claim 13, wherein the sterol is at least one member selected from the group consisting of cholesterol, C₁₂₋₃₁ cholesteryl fatty acid, C₁₂₋₃₁ dihydrocholesteryl fatty acid, polyoxyethylene cholesteryl ether, and polyoxyethylene dihydrocholesteryl ether.

15. The composition according to claim 13, wherein the sterol is at least one member selected from the group consisting of cholesterol, cholesteryl lanolate, cholesteryl oleate, cholesteryl nonanate, cholesteryl macadaminate, and polyoxyethylene dihydrocholesteryl ether.

16. The composition according to claim 13, wherein the sterol is cholesterol.

17. The composition according to claim 13, wherein the cationic lipid is 1,2-dioleoyloxy-3-(trimethylammonium)propane, the amphiphilic glycerophospholipid is an egg-yolk phosphatidylcholine, and the sterol is cholesterol.

18. The composition according to claim 1, wherein a molar ratio of the cationic lipid and the amphiphilic glycerophospholipid is 3:7 to 7:3.

19. The composition according to claim 13, wherein a molar ratio of the cationic lipid and the sterol is 3:7 to 7:3.

20. The composition according to claim 13, wherein a molar ratio of the amphiphilic glycerophospholipid and the sterol is 3:7 to 7:3.

21. The composition according to claim 13, wherein a molar ratio of the cationic lipid and the total of the amphiphilic glycerophospholipid and the sterol is 3:7 to 7:3.

22. The composition according to claim 13, wherein a molar ratio of the cationic lipid, the amphiphilic glycerophospholipid, and the sterol is about 2:1:1.

23. The composition according to claim 1, wherein an average particle diameter of the liposome is 400 nm or more.

24. The composition according to claim 1, wherein an average particle diameter of the liposome is 400 to 1,000 nm.

25. The composition according to claim 1, wherein the liposome is administered to a living body by iontophoresis at a current value of from 0.1 to 0.6 mA/cm².

26. The composition according to claim 1, wherein the active ingredient is selected from the group consisting of a drug, a colorant, a nucleic acid, a protein, an enzyme, a peptide, a lipopolysaccharide, and a cell components.

27. The composition according to claim 1, which is used as medicinal.

28. An electrode structure for administering an active ingredient to a living body by iontophoresis, comprising the composition according to claim 1.

29. An iontophoresis device, comprising the electrode structure according to claim 27.
